# EUROPÄISCHE PATENTSCHRIFT

(11) **EP 2 506 807 B1**
(45) Veröffentlichungstag und Bekanntmachung des Hinweises auf die Patenterteilung: **27.01.2016**
(21) Anmeldenummer: 10784715.4
(22) Anmeldetag: 30.11.2010
(51) Int. Cl.: A61F 2/72, A61F 4/00, A61F 2/68

(54) **ADAPTIVES STEUERUNGS- UND REGELUNGSSYSTEM FÜR PROTHESEN MIT WILLKÜRLICHER STEUERUNG**
ADAPTIVE OPEN-LOOP AND CLOSED-LOOP CONTROL SYSTEM FOR PROSTHESES WITH ARBITRARY CONTROL
SYSTÈME ADAPTATIF DE COMMANDE ET DE RÉGLAGE POUR DES PROTHÈSES AVEC COMMANDE ARBITRAIRE

(30) Priorität: 01.12.2009 DE 102009056466
(43) Veröffentlichungstag der Anmeldung: 10.10.2012
(73) Patentinhaber: Fraunhofer-Gesellschaft zur Förderung der angewandten Forschung e.V., 80686 München (DE)
(72) Erfinder: BUDAKER, Bernhard, 71336 Waiblingen (DE); VON ROSENBERG, Harald, 70569 Stuttgart (DE); KLEINER, Bernhard, 73760 Ostfildern (DE); SCHNEIDER, Urs, 70563 Stuttgart (DE)
(74) Vertreter: Pfenning, Meinig & Partner mbB
(86) Internationale Anmeldenummer: PCT/EP2010/007247
(87) Internationale Veröffentlichungsnummer: WO 2011/066940

(56) Entgegenhaltungen:
- EP-A1- 1 661 543
- US-A- 5 413 611
- US-A1- 2003 023 319
- US-A1- 2003 050 569

## Beschreibung

Die vorliegende Erfindung bezieht sich im Bereich der Konzeption von aktiven und passiven Prothesen auf die Bereitstellung eines Verfahrens zur adaptiven Steuerung und Regelung einer Prothese mit Willkürsteuerung zur nahezu naturgemäßen Bewegung einer Prothese. Die vorliegende Erfindung bezieht sich weiterhin auf ein System zur adaptiven Steuerung und Regelung einer Prothese mit Willkürsteuerung sowie eine Prothese mit Willkürsteuerung.

Seit einiger Zeit wird an Prothesen mit Willkürsteuerung, wobei eine vom Prothesenträger beliebig ausgeführte bzw. willkürliche Aktion zur Manipulation, Beeinflussung oder Ansteuerung einer Prothese gearbeitet. Prothesen mit Willkürsteuerung reagieren beispielsweise auf Muskelkontraktionen direkt mit einer entsprechenden Bewegung. Dazu werden mit Hilfe von verschiedenen Sensoren, beispielsweise von Elektromyografiesensoren, Steuersignale am Muskel selbst abgegriffen und zur Regelung und Steuerung von Aktoren verwendet. Die Aktoren bewirken eine der Muskelkontraktion entsprechende Bewegung der Prothese.

Beispiele für solche Prothesen sind beispielsweise eine Armprothese, wobei das Handgelenk entsprechend der Muskelkontraktion gestreckt oder gebeugt wird, oder Beinprothesen, welche es dem Prothesenträger ermöglichen, sich auf energetisch effiziente Weise fortzubewegen. Eine derartige Prothese wird genauer in der US 5,413,611 A beschrieben.

Prothesen, welche auf Muskelkontraktionen mit entsprechenden Bewegungen reagieren, haben jedoch den Nachteil, dass die Prothese auf gleiche oder ähnliche Muskelkontraktionen in gleicher Art und Weise reagiert, unabhängig davon in welchem Bewegungszustand sich der Prothesenträger befindet. Spannt ein Beinprothesenträger beispielsweise im Sitzen unbewusst eine Muskelpartie an, so kann dies beispielsweise dazu führen, dass das Kniegelenk der Prothese ungewollt gestreckt wird, da diese Bewegung die Folge einer Muskelanspannung im Gehen wäre. Solche unbeabsichtigten Bewegungen der Prothese können eine Gefährdung für den Prothesenträger selbst sowie für seine Umgebung darstellen. Außerdem sind die oben beschriebenen Prothesen mit Willkürsteuerung nicht in der Lage, die Bewegung der Prothese auf Hindernisse, welche vor dem Prothesenträger auftauchen können, abzustimmen. Erkennt der Prothesenträger selbst ein Hindernis, wie beispielsweise eine Türschwelle, nicht rechtzeitig, so besteht für den Prothesenträger die Gefahr zu stolpern.

EP 1 661 543 A1 beschreibt eines tragbaren Handlungsunterstützendes Gerät, dass eine Aktion des Trägers unterstützt oder ausführt. Das Gerät wird dabei über einen Biosignalsensor gesteuert, der ein Biosignal des Trägers detektiert.

US 2003/0050569 A1 beschreibt die Verarbeitung neuronaler Signale und insbesondere ein Verfahren zum Generieren und zum Decodieren der verarbeiteten neuronalen Signalen.

US 2003/0023319 A1 beschreibt ein Prothesensystem, das einen Decodierer zur Vorhersage einer intendierten Handlung verwendet, wie beispielsweise eines Greifens. Die Vorhersage erfolgt dabei aufgrund von verarbeiteten Signalen, die aus einer Messung neuronaler Aktivität erhalten werden. Ausgehend vom Stand der Technik ist es daher die Aufgabe der vorliegenden Erfindung, ein Verfahren sowie ein System zur adaptiven Steuerung und Regelung einer Prothese mit Willkürsteuerung sowie eine Prothese mit Willkürsteuerung zur Verfügung zu stellen, um die Probleme des Standes der Technik zu beseitigen. Die Aufgabe der vorliegenden Erfindung besteht also darin, die Bewegung der Prothese nicht nur an die Muskelaktivität des Prothesenträgers anzupassen, sondern an den Bewegungszustand des Prothesenträgers anzupassen. Weiterhin soll die vorliegende Erfindung das Sturzrisiko des Prothesenträgers aufgrund von Hindernissen verringern und die Bewegung der Prothese möglichst gut an die durch unterbewusstes Verhalten beeinflusste Bewegung eines entsprechenden gesunden Körperteils annähern.

Die Aufgabe der vorliegenden Erfindung wird durch das Verfahren zur adaptiven Steuerung und Regelung einer Prothese mit Willkürsteuerung nach Anspruch 1, das adaptive Steuerungs- und Regelsystem für Prothesen mit Willkürsteuerung nach Anspruch 7 sowie die Prothese mit Willkürsteuerung nach Anspruch 12 gelöst. Vorteilhafte Weiterbildungen der vorliegenden Erfindung werden in den jeweiligen abhängigen Ansprüchen gegeben.

Erfindungsgemäß wird zur adaptiven Steuerung und Regelung einer Prothese mit Willkürsteuerung zunächst an mindestens einem Muskel des Prothesenträgers eine Vielzahl an Muskelaktivitätssignalen gemessen. Als Muskel des Prothesenträgers wird vorzugsweise ein oder mehrer Muskeln, welche sich im Nachbarbereich des durch die Prothese ersetzten Körperteils befinden, gewählt, d.h. beispielsweise bei Fuß- oder Beinprothesen ein oder mehrere Muskeln am Fuß oder Bein und bei Hand- bzw. Armprothesen ein oder mehrere Muskeln an Hand oder Arm.

Gleichzeitig zur Messung der Muskelaktivitätssignale werden Zustandsinformationen zu einem tatsächlichen Bewegungszustand des Prothesenträgers gemessen, aus welchen mindestens ein möglicher, aktueller Bewegungszustand des Prothesenträgers bestimmt wird. Als tatsächlicher Bewegungszustand des Prothesenträgers kann eine der folgenden Bewegungsformen bzw. Bewegungszustände in Frage kommen: Sitzen, Stehen, Gehen, Laufen, Rennen, Springen, Knien, Hinsetzen, Hinlegen, Aufrichten, Aufstehen, Bücken, Zugreifen, Festhalten, Loslassen, Werfen oder Schubsen, wobei insbesondere die Zustände Sitzen bis Bücken für Fuß- bzw. Beinprothesen und die Zustände Zugreifen bis Schubsen für Hand- bzw. Armprothesen relevant sind. Allgemein können sämtliche für aktive und passive Prothesen relevante Bewegungsformen und/oder Bewegungszustände als möglicher aktueller Bewegungszustand verstanden werden.

Nach der Messung der Muskelaktivitätssignale und der Zustandsinformationen sowie der Bestimmung des möglichen aktuellen Bewegungszustands werden zumindest aus den Muskelaktivitätssignalen mit Hilfe eines Verfahrens zur Detektion von Signalmustern Muskelaktivitätsmerkmale extrahiert. Gegebenenfalls kann bei der Extraktion von Muskelaktivitätsmerkmalen auch der mindestens eine mögliche aktuelle Bewegungszustand berücksichtigt werden.

Anschließend an die Extraktion der Muskelaktivitätsmerkmale wird erfindungsgemäß aus den extrahierten Muskelaktivitätsmerkmalen mit Hilfe des mindestens einen möglichen, aktuellen Bewegungszustands mindestens ein Willkürsignal bestimmt. Die Bestimmung des Willkürsignals kann beispielsweise mit Hilfe eines Klassifizierungsverfahrens durchgeführt werden.

Das aus den Muskelaktivitätssignalen und den Zustandsinformationen bestimmte Willkürsignal dient der Auswertung und/oder der Regelung sowie Steuerung einer Aktorik der Prothese, durch die eine Veränderung der Protheseneigenschaften, insbesondere eine Bewegung der Prothese, welche bevorzugt eine Relativbewegung von zwei über ein Gelenk verbundenen Partien der Prothese ist, oder eine Veränderung der Dämpfungseigenschaften der Prothese, ausgelöst wird.

Das erfindungsgemäße Verfahren stellt aufgrund der Bestimmung des Willkürsignals aus den extrahierten Muskelaktivitätsmerkmalen sowie dem mindestens einen möglichen aktuellen Bewegungszustands die Anpassung der Bewegung der Prothese auf den jeweiligen Bewegungszustand sicher. Dem Prothesenträger wird so die Sicherheit gegeben, dass die Prothese aufgrund von unbewussten und/oder ungewollten Muskelanspannungen keine für seinen tatsächlichen Bewegungszustand unnatürliche Bewegung ausführt. Das Risiko einer Gefährdung des Prothesenträgers und/oder seiner Umgebung aufgrund von für seinen tatsächlichen Bewegungszustand unnatürlichen Bewegungen wird also minimiert. Außerdem wird der Rechenaufwand für die Klassifizierung der Muskelaktivitätsmerkmale durch die Berücksichtigung des möglichen aktuellen Bewegungszustands bei der Klassifizierung verringert und vereinfacht. Im Gegensatz zu herkömmlichen Prothesen, welche lediglich eine manuelle Einstellung des Bewegungszustands zulassen, besteht ein weiterer Vorteil des erfindungsgemäßen Verfahrens in einer automatischen Modusumschaltung, d.h. in einer automatischen Anpassung an verschiedene Bewegungsabläufe bzw. Bewegungszustände.

Die Bestimmung des Willkürsignals mit dem erfindungsgemäßen Verfahren erfolgt vorteilhafterweise in Echtzeit, dass die Bewegung der Prothese rechtzeitig aufgelöst wird.

Im Falle des erfindungsgemäßen Verfahrens ist der mindestens eine mögliche aktuelle Bewegungszustand vorzugsweise als ein Bewegungszustand definiert, welcher mit einer einen Schwellwert überschreitenden Wahrscheinlichkeit einem tatsächlichen Bewegungszustand des Prothesenträgers entspricht. Der Schwellwert kann dabei 0 % oder mehr, vorzugsweise 25 % oder mehr, betragen oder auch bei noch höheren Werten liegen. Bei einem Schwellwert von 0% werden alle Bewegungszustände, welche dem tatsächlichen Bewegungszustand in einem Parameter bzw. einer Zustandsinformation ähneln, berücksichtigt. Wenn beispielsweise der Parameter "Körper in senkrechter Haltung" bestimmt ist, kann der tatsächliche Bewegungszustand Gehen, Stehen oder Rennen etc. sein.

Wie bereits erwähnt, kann der Bewegungszustand bereits bei der Merkmalsextraktion eine Rolle spielen, da je nach Bewegungszustand andere Muskelaktivitätsmerkmale gewonnen werden bzw. sich ihre Gewichtung verändert. Nach der Messung von Zustandsinformationen werden vorzugsweise Wahrscheinlichkeitsverteilungen für verschiedene definierte Bewegungszustände als mögliche aktuelle Bewegungszustände ermittelt.

Für die Bewegungszustände, die mit einer Wahrscheinlichkeit größer einem Schwellwert erkannt wurden, kann dann ein entsprechender Satz an Merkmalen aus dem Eingangsdatenstrom bzw. den Muskelaktivitätssignalen bestimmt werden, welcher mit der jeweils zugehörigen Bewegungszustands-Wahrscheinlichkeit an einen Klassifikator weitergeleitet werden kann. Es kann also sowohl Wahrscheinlichkeiten für den erkannten Zustand als auch Wahrscheinlichkeiten für das erkannte Willkürsignal in jedem dieser Bewegungszustände geben. Die anschließende Auswertung und Bestimmung des Willkürsignals kann dabei entweder nur den Bewegungszustand mit der höchsten Wahrscheinlichkeit verwenden, und für diesen die Wahrscheinlichkeit der Willkürerkennung bestimmen, oder es können alle Bewegungszustands-Wahrscheinlichkeiten bis zur Bestimmung des Gesamt-Willkürsignals weiterverarbeitet werden.

Wird die Bestimmung der Muskelaktivitätsmerkmale also aktiv durch den mindestens einen möglichen aktuellen Bewegungszustand beeinflusst, so führt dies zu einer verbesserten Abstimmung der Bewegung der Prothese auf den Bewegungszustand des Prothesenträgers und verringert wiederum den Rechenaufwand zur Bestimmung des Willkürsignals, wodurch die Laufzeit verändert wird.

Vorzugsweise werden die Muskelaktivitätssignale nicht direkt für die Extraktion von Muskelaktivitätsmerkmalen verwendet, sondern zunächst miteinander zu virtuellen Muskelaktivitätssignalen kombiniert. Durch die Bestimmung virtueller Muskelaktivitätssignale kann beispielsweise die Anordnung einzelner Sensoren der ersten Erkennungseinheit, insbesondere die Sensorarrayauslegung, flexibler gestaltet werden. Außerdem können mit Hilfe der virtuellen Muskelaktivitätssignale Störungen auf einzelnen Kanälen erkannt und gegebenenfalls eliminiert werden. Des Weiteren kann ein Ausfall oder eine Störung einzelner Sensoren, insbesondere einzelner Elektroden, bzw. eine fehlerhafte Anordnung einzelner Sensoren, beispielsweise mit der Folge eines schlechten, unzureichenden Kontakts zwischen Sensor bzw. Elektrode und Haut des Probenträgers, erkannt und gegebenenfalls durch Ersetzen einzelner Sensoren bzw. geeigneter Anordnung oder entsprechende Beachtung bei der Berechnung beseitigt werden. Der Einsatz von virtuellen Muskelaktivitätssignalen führt außerdem zu einer Erhöhung der Redundanz.

Vorzugsweise wird nach der Extraktion von Muskelaktivitätsmerkmalen eine Dimensionsreduktion durchgeführt.

Die Vielzahl an Muskelaktivitätssignalen wird vorzugsweise mittels mindestens einer ersten Erkennungseinheit zur Messung von Muskelaktivitätssignalen gemessen. Als mindestens eine erste Erkennungseinheit kann beispielsweise eine eine oder mehrere Elektroden aufweisende erste Erkennungseinheit verwendet werden. Wird die Messung mit einer mehrere Elektroden aufweisenden ersten Erkennungseinheit durchgeführt, so sind die Elektroden vorzugsweise als räumliche und verteilte Elektrodenanordnungen mit zweidimensionaler räumlicher Ausdehnung und Verteilung und unterschiedlicher Geometrie, welche eine Sensormatrix bilden kann, beispielsweise in Gitterform, angeordnet. Als Elektroden können beispielsweise die Elektroden eines Elektromyografen, eines Elektroenzephaolgrafen oder eines Elektroneurografen eingesetzt werden.

Alternativ oder zusätzlich kann mindestens einen Drucksensor oder eine räumliche und verteilte Drucksensoranordnung als erste Erkennungseinheit verwendet werden. Vorteilhafterweise enthält die zur Messung von Muskelaktivitätssignalen eingesetzte erste Erkennungseinheit mindestens eine Elektrode oder eine Elektrodenanordnung sowie mindestens einen Drucksensor oder mindestens eine Drucksensoranordnung, wobei je eine Elektrode oder je eine Elektrodenanordnung und je ein Drucksensor oder je eine Drucksensoranordnung eine funktionale Einheit bilden.

Mit Hilfe der Drucksensorik können folgende Drücke gemessen werden, wobei eine beliebige Kombination innerhalb dieser Druckmessungen möglich ist:
- Druck der ersten Erkennungseinheit, beispielsweise eines einzelnen Sensors, insbesondere einer Elektrode, auf die Haut des Prothesenträgers (Kontaktpressung der Einzelelektrode),
- Druck der ersten Erkennungseinheit, insbesondere eines ganzen Sensor-Arrays, bzw. Sensor-Gitters gegen die Prothese bzw. einen Prothesenschaft (gemittelter Druck),
- Druck von Prothese gegen einen die Umgebung, beispielsweise von Ferse und/oder Zeh an Bein- bzw. Fußprothese gegen den Untergrund, wobei die Messung über zwei diskrete Drucksensoren erfolgt.

Als mindestens eine erste Erkennungseinheit kann anstelle oder zusätzlich zu Elektroden und/oder Drucksensoren auch mindestens ein akustischer Sensor, insbesondere einen Ultraschallsensor bzw. Ultraschallwandler, oder ein anderer Sensor zur nicht-invasiver Erkennung und Messung von Muskelkontraktionen verwendet werden.

Zur Messung von Muskelaktivitätssignalen werden vorzugsweise mehrere erste Erkennungseinheiten, welche gleich oder unterschiedlich ausgebildet sein können, eingesetzt, um die Bewegung der Prothese genauer und natürlicher steuern zu können.

Zur Messung der Zustandsinformationen zum tatsächlichen Bewegungszustand des Prothesenträgers wird vorzugsweise eine zweite Erkennungseinheit zur Messung von Zustandsinformationen zum Bewegungszustand des Prothesenträgers eingesetzt. Als zweite Erkennungseinheit wird vorzugsweise mindestens ein Inertialsensor, insbesondere mindestens ein Translationssensor bzw. Beschleunigungssensor und/oder mindestens ein gyroskopischer Sensor bzw. DrehratenSensor verwendet. Die Messung mittels Inertialsensor ermöglicht die Bestimmung des Bewegungszustandes im Raum anhand von bis zu sechs Freiheitsgraden. Alternativ oder zusätzlich zum mindestens einen Inertialsensor können die Zustandsinformationen mit mindestens einem Magnetfeldsensor und/oder mindestens einem Rotationssensor und/oder mindestens einem Trägheitssensor gemessen werden.

Zur Messung von Zustandsinformationen werden vorzugsweise mehrere zweite Erkennungseinheiten, welche gleich oder unterschiedlich ausgebildet sein können, eingesetzt, um die Qualität und Richtigkeit der Zustandsinformationen zu verbessern.

Das erfindungsgemäße Verfahren sieht vorzugsweise vor, während der Messung von Muskelaktivitätssignalen und von Zustandsinformationen zusätzlich mindestens ein Umgebungssignal aufzunehmen, und damit ein näheres Umfeld bzw. Terrain der Prothese bzw. des Prothesenträgers zu erfassen. Als näheres Umfeld der Prothese kann ein Bereich im Umkreis von 10 m um die Prothese verstanden werden, wobei ein Bereich von 0 m bis 10 m als Fernfeld und ein Bereich von 0 m bis 2 m als Nahfeld definiert ist. Vorzugsweise ist das nähere Umfeld der Prothese ein Umkreis von drei bis vier Gangzyklen um die Prothese herum, wobei ein Gangzyklus als Doppelschritt bzw. als Bewegungsablauf von einem ersten Aufsetzen der Prothese auf den Untergrund über ein Abrollen des Fußes und eine anschließende Bewegung der Prothese durch die Luft über dem Untergrund zu einem nachfolgenden Aufsetzten der Prothese zu verstehen ist.

Im Falle einer Fuß- und/oder Beinprothese kann mit Hilfe des Umgebungssignals beispielsweise ein Modell zur Beschaffenheit des Untergrunds, insbesondere ein dreidimensionales Modell des Untergrunds, erstellt werden, indem beispielsweise Steigungsschwankungen, d.h. Steigungen und Gefälle, des Untergrunds, Unebenheiten, Löcher, Treppen bzw. Stufen, Hindernisse und/oder die Oberflächenbeschaffenheit des Untergrunds berücksichtigt werden. Zur Steuerung und Regelung einer Hand- und/oder Armprothese dagegen können mit Hilfe des Umgebungssignals beispielsweise Gegenstände, welche gegriffen werden sollen, analysiert werden, um eine Zerstörung oder Verletzung des Gegenstandes beim Greifen zu vermeiden. Das Umgebungssignal, welches zur Steuerung und Regelung von Armprothesen gemessen wird, kann beispielsweise Informationen über den Abstand beispielsweise des Unterarms zu einem Hindernis abschätzen, um beispielsweise beim Ausstrecken des Armgelenks eine Kollision des Unterarms mit dem Hindernis zu vermeiden. Bei Armprothesen kann das Umgebungssignal auch Aufschluss über den Abstand zu einer zu begrüßenden Person geben, um beispielsweise den Streckungswinkel eines Prothesengelenks beim Handschlag geeignet anzupassen.

Vorzugsweise wird das mindestens eine Umgebungssignal mit Hilfe mindestens einer dritten Erkennungseinheit zur Erfassung mindestens eines Umgebungssignals gemessen. Als dritte Erkennungseinheit können mindestens ein Nah- und/oder Fernbereichsensorsystem eingesetzt werden. Beispielsweise kann mindestens ein akustischer Sensor, insbesondere ein Ultraschallsensor, mindestens ein optischer Sensor und/oder mindestens ein scannender Sensor eingesetzt werden. Außerdem oder zusätzlich kann das Umgebungssignal auch mit Hilfe mindestens einer Kamera, insbesondere eines einfachen Kamerasensors und/oder eines 3D-Kamerasensors, wie beispielsweise einem Fotomischdetektor, als Teil der dritten Erkennungseinheit aufgenommen werden. Des weiteren besteht die Möglichkeit, ein Umgebungssignal mit Hilfe mindestens eines Laserdistanzsensors und/oder mindestens eines Nahbereichradars und/oder mindestens eines Projektors als Teil der dritten Erkennungseinheit zu messen.

Vorteilhafterweise wird das Umgebungssignal mit Hilfe mehrere dritter Erkennungseinheiten, welche gleich oder unterschiedlich ausgebildet sein können, aufgenommen, um die Verlässlichkeit der im Umgebungssignal enthaltenen Informationen zu verbessern.

Mit Hilfe des Umgebungssignals kann das Willkürsignal, was aus den Muskelaktivitätssignalen und den Zustandsinformationen bestimmt wurde, ergänzt werden. Das Willkürsignal wird vorzugsweise mit Hilfe des mindestens einen Umgebungssignals gefiltert und/oder bewertet, wobei vorzugsweise mindestens ein Filter zur Bewertung des Willkürsignals verwendet wird.

Somit wird dem Prothesenträger ein Verfahren zur Steuerung und Regelung seiner Prothese zu Verfügung gestellt, welches sein Sturzrisiko verringert und ihm somit ein sichereres Gefühl bei seiner Bewegung gibt. Die vorliegende Erfindung bezieht sich weiterhin auf ein adaptives Steuerungs- und Regelsystem für Prothesen mit Willkürsteuerung. Ein erfindungsgemäßes adaptives Steuerungs- und Regelungssystem weist ein Signalbestimmungssystem, ein Regelungssystem und ein Steuersystem auf. Das Signalbestimmungssystem ist zur Bestimmung eines Willkürsignals, welches durch eine vom Prothesenträger beliebig ausgeführte Aktion ausgelöst ist, ausgebildet. Das Regelungssystem ist so beschaffen, dass eine Auswertung und/oder eine Regelung einer Aktorik einer Prothese zur Veränderung der Protheseneigenschaften, insbesondere der Bewegung oder der Dämpfungseigenschaft der Prothese, mit Hilfe des Willkürsignals durchgeführt werden kann und ein Regelsignal ausgegeben werden kann. Das Steuerungssystem zuletzt dient der Ansteuerung der Aktorik der Prothese auf der Basis des Regelsignals.

Erfindungsgemäß weist das Signalbestimmungssystem mindestens eine erste Erkennungseinheit zur Messung einer Vielzahl an Muskelaktivitätssignalen an einem Muskel des Prothesenträgers, mindestens eine zweite Erkennungseinheit zur Messung von Zustandsinformationen zu einem tatsächlichen Bewegungszustand des Prothesenträgers sowie mindestens einen Prozessor zur Bestimmung von Muskelaktivitätsmerkmalen mit Hilfe eines Verfahrens zur Detektion von Signalmustern auf. Der Prozessor ist dabei derart ausgebildet, dass aus den Zustandsinformationen mindestens ein möglicher, aktueller Bewegungszustand des Prothesenträgers bestimmt werden kann und zumindest aus den Muskelaktivitätssignalen Muskelaktivitätsmerkmale extrahiert werden können, aus denen mit Hilfe des mindestens einen möglichen, aktuellen Bewegungszustands das mindestens eine Willkürsignal, vorzugsweise in Echtzeit, bestimmt werden kann.

Das erfindungsgemäße System zur adaptiven Steuerung einer Prothese ermöglicht dem Prothesenträger die Vermeidung von für seinen Bewegungszustand unnatürlichen Bewegungen der Prothese und die damit verbundene Gefährdung von sich selbst und/oder seiner Umgebung. Der Prothesenträger kann sich also besser die Bewegung seiner Prothese besser abschätzen und muss weniger Konzentration auf die Vermeidung fehlerhafter Bewegungen aufwenden. Außerdem ist der Rechenaufwand zur Bestimmung des Willkürsignals aufgrund der Berücksichtigung des Bewegungszustands verringert. Ein weiterer Vorteil des erfindungsgemäßen Systems besteht in einer automatischen Modusumschaltung, d.h. in einer automatischen Anpassung an verschiedene Bewegungsabläufe bzw. Bewegungszustände.

Vorzugsweise ist der Prozessor so ausgebildet, dass bereits bei der Extraktion von Muskelaktivitätsmerkmalen aus den Muskelaktivitätssignalen der mindestens eine mögliche aktuelle Bewegungszustand des Prothesenträgers berücksichtigt wird, um den Rechenaufwand bei der Extraktion der Muskelaktivitätsmerkmale zu verringern.

Der Prozessor kann auch derart beschaffen sein, dass vor der Extraktion von Muskelaktivitätsmerkmalen mehrere Muskelaktivitätssignale miteinander zu einem virtuellen Muskelaktivitätssignal kombiniert werden.

Die mindestens eine erste Erkennungseinheit zur Messung einer Vielzahl an Muskelaktivitätssignalen an einem Muskel eines Prothesenträgers kann beispielsweise eine oder mehrere Elektroden enthalten, welche Elektroden eines Elektromyografen, eines Elektroenzephaolgrafen und/oder eines Elektroneurografen sein können. Vorteilhafterweise sind mehrere Elektroden zu mindestens einer räumlichen und verteilten Elektrodenanordnung mit unterschiedlicher Geometrie, welche eine Sensormatrix bilden kann, angebracht sind. Als Elektrodenanordnung kann ein Elektrodengitter gewählt sein.

Die erste Erkennungseinheit kann außerdem oder alternativ einen oder mehrere Drucksensoren aufweisen. Auch die Drucksensoren können als räumliche und verteilte Drucksensoranordnung mit beliebiger Geometrie angeordnet sein. Vorzugsweise ist die mindestens eine erste Erkennungseinheit eine funktionale Einheit aus mindestens einer Elektrode oder mindestens einer Elektrodenanordnung und mindestens einem Drucksensor oder mindestens einer Drucksensoranordnung.

Die erste Erkennungseinheit kann zusätzlich oder alternativ mindestens einen Ultraschallsensor und/oder mindestens eine alternative Sensoranordnung, welche eine Muskelkontraktion nicht-invasiv erkennen und messen kann, aufweisen.

Die mindestens eine erste Erkennungseinheit ist vorzugsweise derart an einer Prothese angeordnet, dass die erste Erkennungseinheit in direktem Kontakt mit der Haut des Prothesenträgers im Bereich eines Muskels, dessen Aktivierung gemessen werden soll, steht. Bei Fuß- und/oder Beinprothesen ist die mindestens eine erste Erkennungseinheit bevorzugt im Übergangsbereich zwischen Prothese und Unterschenkel- oder Oberschenkelstumpf des Prothesenträgers angeordnet, während bei Hand- oder Armprothesen die erste Erkennungseinheit vorzugsweise im Übergangsbereich zwischen Prothese und Unterarm bzw. Oberarmstummel angeordnet ist.

Das erfindungsgemäße Steuerungs- und Regelsystem weist vorzugsweise mehrere erste Erkennungseinheiten auf, welche an unterschiedlichen Muskelpartien des Prothesenträgers angeordnet sind, wobei die an unterschiedlichen Muskelpartien gemessenen Muskelaktivitäten letztlich eine Vielzahl an möglichen Bewegungen der Prothese bewirken. Je mehr Muskelaktivitätssignale gemessen werden, desto genauer bzw. präziser und natürlicher kann die Bewegung der Prothese geregelt und gesteuert werden.

Die mindestens eine zweite Erkennungseinheit des erfindungsgemäßen Steuerungs- und Regelsystems kann als mindestens ein Inertialsensor, insbesondere mindestens ein Translationssensor bzw. Beschleunigungssensor und/oder als mindestens ein gyroskopischer Sensor bzw. Drehratensensor ausgebildet sein. Ein Inertialsensor ermöglicht die Bestimmung des Bewegungszustands des Prothesenträgers im Raum mit Hilfe von bis zu sechs, insbesondere genau sechs, Freiheitsgraden. Die zweite Erkennungseinheit kann weiterhin oder alternativ einen Magnetfeldsensor und/oder mindestens einen Rotationssensor und/oder mindestens einen Trägheitssensor aufweisen.

Die mindestens eine zweite Erkennungseinheit ist vorzugsweise an einer geeigneten Stelle in die Prothese integriert, so dass sie vor Beschädigungen von außen geschützt ist.

Der Prozessor des Steuerungs- und Regelsystems enthält vorzugsweise eine Vorverarbeitungseinheit, eine Merkmalsextraktionseinheit sowie eine Klassifizierungseinheit. Die Vorverarbeitungseinheit dient vorteilhafterweise der Erzeugung mindestens eines virtuellen Muskelaktivitätssignals aus einer Vielzahl an Muskelaktivitätsdaten. Weiterhin kann die Vorverarbeitungseinheit zur Bestimmung des mindestens einen möglichen, aktuellen Bewegungszustands des Prothesenträgers aus den Zustandsinformationen vorgesehen sein. Als mindestens ein möglicher, aktueller Bewegungszustand ist dabei insbesondere der Bewegungszustand zu verstehen, welcher mit einer einen Schwellwert überschreitenden Wahrscheinlichkeit dem tatsächlichen Bewegungszustand des Prothesenträgers entspricht, wobei der Schwellwert 0 % oder mehr, insbesondere 25% oder mehr, betragen kann.

Die Merkmalsextraktionseinheit dient vorzugsweise der Gewinnung von Muskelaktivitätsmerkmalen aus dem mindestens einen virtuellen Muskelaktivitätssignal oder aus der Vielzahl an Muskelaktivitätssignalen. Vorzugsweise ist die Merkmalsextraktionseinheit derart ausgebildet, dass bei der Extraktion von Muskelaktivitätsmerkmalen der mindestens eine mögliche, aktuelle Bewegungszustand des Prothesenträgers berücksichtigt wird.

Die Klassifizierungseinheit des Prozessors ist vorteilhafterweise derart ausgebildet, dass die Muskelaktivitätsmerkmale mit Hilfe des mindestens einen möglichen, aktuellen Bewegungszustandes in Klassen eingeteilt werden können und ein Willkürsignal bestimmt werden kann.

Der Prozessor ist vorzugsweise in der Prothese integriert und direkt über Kabel oder sonstige kabellose Übertragungsarten mit den Erkennungseinheiten und der Aktorik verbunden. Alternativ kann der Prozessor auch als externe Einheit ausgebildet sein, die vorzugsweise vom Prothesenträger mitgeführt wird und über Kabel oder kabellos mit den Erkennungseinheiten und der Aktorik verbunden ist.

Um die Bewegung der Prothese mit Willkürsystem auf die Umgebungsgegebenheiten abstimmen zu können, weist das erfindungsgemäße Steuerungs- und Regelsystem vorteilhafterweise als Teil des Bestimmungssystems mindestens eine dritte Erkennungseinheit zur Erfassung mindestens eines Umgebungssignals auf. Dabei kann die dritte Erkennungseinheit beispielsweise mindestens ein Nah- und/oder Fernbereichsensor enthalten. Insbesondere kann die dritte Erkennungseinheit mindestens einen akustischen Sensor, insbesondere einen Ultraschallsensor, einen optischen Sensor und/oder einen scannenden Sensor aufweisen. Alternativ oder zusätzlich kann die dritte Erkennungseinheit auch eine Kamera, insbesondere einen einfachen Kamerasensor oder einen 3D-Kamerasensor, beispielsweise einen PMD, enthalten. Die dritte Erkennungseinheit kann außerdem oder alternativ mindestens einen Laserdistanzsensor und/oder mindestens einen Nahbereichradar und/oder mindestens einen Projektor aufweisen. Das mindestens eine Umgebungssignal dient der Filterung und Bewertung des Willkürsignals, welches aus den Muskelaktivitätssignalen sowie den Zustandsinformationen bestimmbar ist.

Zur Verbesserung des Umgebungssignals weist das erfindungsgemäße System vorzugsweise mehrere, gleich oder unterschiedlich ausgebildete dritte Erkennungseinheiten auf. Die dritte Erkennungseinheit ist vorteilhafterweise in eine Prothese integriert, wobei sie vor äußeren Einflüssen, wie Verschmutzung und Verkratzen, geschützt sein sollte. Alternativ kann auch mindestens eine der dritten Erkennungseinheiten vom Prothesenträger als externe Einheit mitgeführt werden.

Je nach Art der Prothese wird ein näheres Umfeld der Prothese und/oder des Prothesenträgers, insbesondere ein Bereich im Umkreis von 10 m, insbesondere ein Fernfeld von 0 m bis 10 m und/oder ein Nahfeld von 0 m bis 2 m, insbesondere ein Bereich im Umkreis von drei bis vier Gangzyklen um die Prothese herum, wobei ein Gangzyklus als Doppelschritt bzw. als Bewegungsablauf von einem ersten Aufsetzen der Prothese auf den Untergrund über ein Abrollen des Fußes und eine Bewegung der Prothese durch die Luft über dem Untergrund zu einem nachfolgenden Aufsetzten der Prothese zu verstehen ist, gemessen. Dabei werden vorzugsweise die Beschaffenheit des Untergrunds, z.B. Steigungen, Stufen und/oder Unebenheiten, oder Hindernisse im Raum beachtet. Beispielsweise bei Beinprothesen wird entsprechend der Umgebung die Grundwinkeleinstellung angepasst.

Das Steuerungs- und Regelsystem weist vorzugsweise einen Filter zur Bewertung des Willkürsignals mit Hilfe des mindestens einen Umgebungssignals auf. Das gefilterte Willkürsignal enthält dann nicht nur Informationen über die vom Prothesenträger aktivierte Bewegung der Prothese, sondern auch Informationen zur Beschaffenheit der Umgebung, so dass die Bewegung der Prothese auch an die Umgebung bzw. der Umfeld der Prothese und/oder des Prothesenträgers angepasst ist. Die Berücksichtigung des Umgebungssignals bzw. der Umgebungsbeschaffenheit dient insbesondere als eine Art Sicherheitssystem des Steuerungs- und Regelsystems.

Das erfindungsgemäße System weist als Energieversorgung vorzugsweise mindestens eine Batterie oder mindestens einen Akkumulator auf, welcher aus einem Energierückgewinnungssystem der Prothese gespeist werden kann.

Die vorliegende Erfindung betrifft weiterhin eine Prothese mit Willkürsteuerung, mit deren Hilfe ein Prothesenträger aktive Bewegungen ausführen kann, wobei die Prothese ein adaptives Steuerungs- und Regelsystem, wie es oben näher beschrieben wurde, und eine Aktorik aufweist.

Die Prothese kann dabei als aktive oder passive Prothese ausgebildet sein. Vorzugsweise ist die Prothese eine Exoprothese und ist vorzugsweise als Bein-, Fuß-, Arm- oder Handprothese ausgebildet.

Je nach Art der Prothese ist die Aktorik dazu ausgebildet, zwei über ein Gelenk miteinander verbundene Glieder der Prothese relativ zueinander zu bewegen (aktive Prothese) und/oder die Dämpfungseigenschaften der Prothese zu beeinflussen (passive und auch aktive Prothese). Außerdem kann die Aktorik dafür sorgen, dass ein Winkel- oder Bewegungsbereich eines Gelenks freigegeben und/oder durch eine Sperreinrichtung arretiert wird.

Die Aktorik der erfindungsgemäße Prothese ist vorzugsweise als mindestens einen Aktor und/oder mindestens einen Motor, welche die Bewegung eines Gelenkes realisieren, und/oder mindestens ein Dämpfungselement, welches die Dämpfungseigenschaften einer passiven oder aktiven Prothese beeinflusst, ausgebildet. Außerdem kann die Prothese mindestens eine Rückmeldeeinheit an den Prothesenträger (Feedback) enthalten, welche dem Prothesenträger anzeigen, dass eine Bewegung der Prothese ausgelöst wurde.

Nachfolgend werden einige Beispiele für erfindungsgemäße Verfahren und Systeme zur Regelung und Steuerung von Prothesen mit Willkürsteuerung gegeben.

Es zeigen:
- Figur 1: ein Grundsystem zur Detektion der Muskelaktivität;
- Figur 2: ein Blockdiagramm zur Willkürsignal- und Bewegungszustandserkennung;
- Figuren 3A und 3B: ein Flussdiagramm zur Erzeugung eines Willkürsignals; und
- Figur 4: ein erfindungsgemäßes Steuerungs- und Regelsystem.

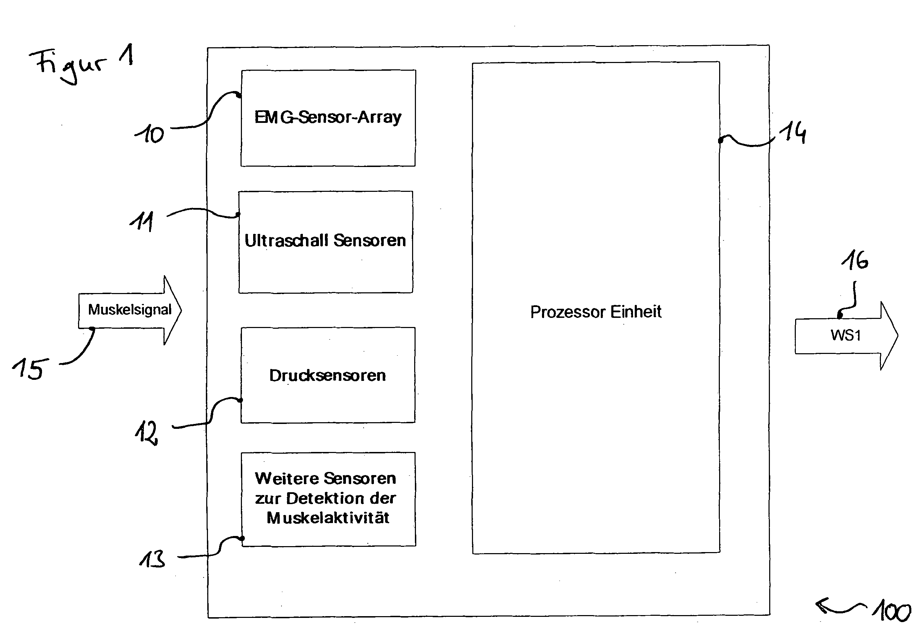

Figur 1 zeigt ein System 100 zur Erzeugung eines Willkürsignals aus Muskelaktivitätssignalen. Das System 100 weist Elektromyografiesensorgitter 10, Ultraschallsensoren 11, Drucksensoren 12 und/oder weitere Sensoren 13 zur Detektion der Muskelaktivität sowie einen Prozessor 14 auf. Ein Muskel, welcher in irgendeiner Weise belastet wird (nicht gezeigt) gibt ein Muskelsignal 15 aus. Dieses Muskelsignal 15 kann mit Hilfe der Elektromyografiesensorgitter 10, Ultraschallsensoren 11, Drucksensoren 12 und/oder weiteren Sensoren 13 zur Detektion der Muskelaktivität gemessen werden und in Form eines Muskelaktivitätssignals an die Prozessoreinheit 14 geleitet werden. Die Prozessoreinheit 14 sorgt für eine Bestimmung eines Willkürsignals 16 aus dem Muskelaktivitätssignal.

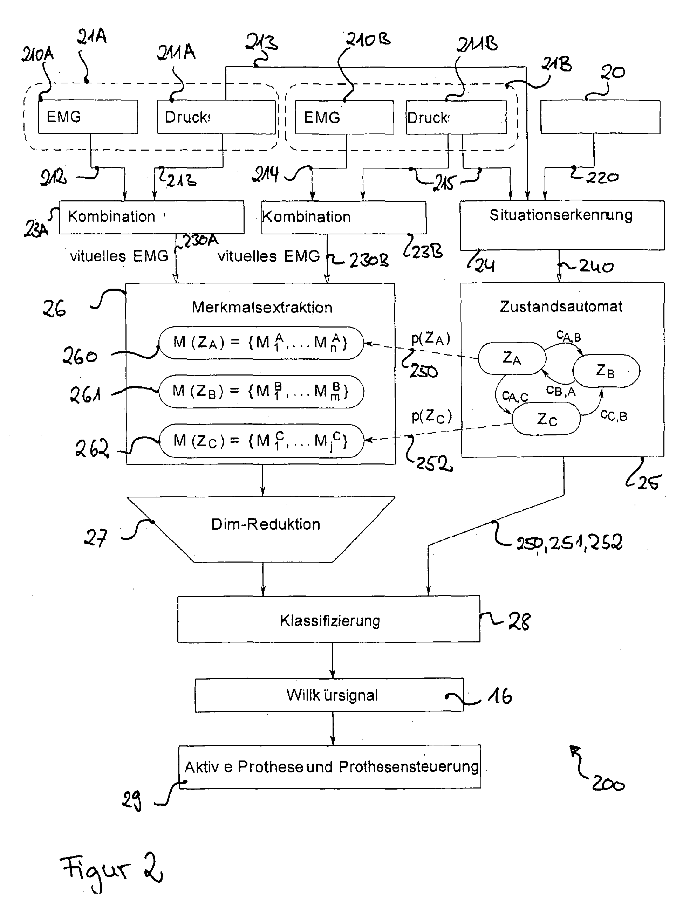

Figur 2 zeigt den schematischen Aufbau 200 einer Erkennungslogik für das Willkürsignal. Die Erkennungslogik kann mit dem System 100 aus Figur 1 erfolgen.

In einen ersten Schritt werden in verschiedenen Mess-Ebenen 21A, 21B an einem oder mehreren Muskeln eines Prothesenträgers Elektromyographie-Signale (EMG-Signale) 212, 214 (Elektromyographie-Messung 210A, 210B) und Drucksignale 213, 215 gemessen (Druckmessung 211A, 211B). Gleichzeitig werden die mit einem Inertialsensor aufgenommenen Daten 220 zum tatsächlichen Bewegungszustand des Prothesenträgers aufgenommen (Inertialmessung 20).

In einem zweiten Schritt werden die in der Ebene 1A gemessenen EMG-Signale 212 und Drucksignale 213 miteinander zu virtuellen EMG-Signalen 230A kombiniert (Kombination 23A). Entsprechend werden die in der Ebene 21B gemessenen Signale 214 und 215 miteinander zu virtuellen EMG-Signalen 230B kombiniert (Kombination 23B). Zeitgleich werden die Daten 220 und die Drucksignale 213, 215 der beiden Ebene 21A und 21B miteinander zu Zustandsinformationen 240 verrechnet (Situationserkennung 24). Aus den Zustandinformationen 240 werden dann Wahrscheinlichkeiten 250, 251, 252 für mögliche, aktuelle Bewegungszustände des Prothesenträgers, wie beispielsweise Sitzen, Stehen, Gehen, Laufen, u.ä., berechnet und eine Voraussage (Prädiktion) der Bewegungssituation erstellt (Zustandsautomat 25).

Anschließend werden in einem dritten Schritt aus den virtuellen EMG-Signalen 230A und 230B unter Berücksichtigung der Wahrscheinlichkeiten 250, 251, 252 für mögliche, aktuelle Bewegungszustände Muskelaktivitätsmerkmale 260, 261, 262 extrahiert (Merkmalsextraktion 26), welche anschließen eine Dimensionsreduktion 27 durchlaufen und in dimensionsreduzierten Muskelaktivitätsmerkmale umgewandelt werden.

In einem vierten Schritt erfolgt dann eine Klassifizierung 28 der dimensionsreduzierten Muskelaktivitätsmerkmale mit Hilfe der Wahrscheinlichkeiten 250, 251, 252 für mögliche, aktuelle Bewegungszustände zur Bestimmung eines Willkürsignals 16, welches der Regelung und Steuerung 29 der zu bewegenden Prothese dient.

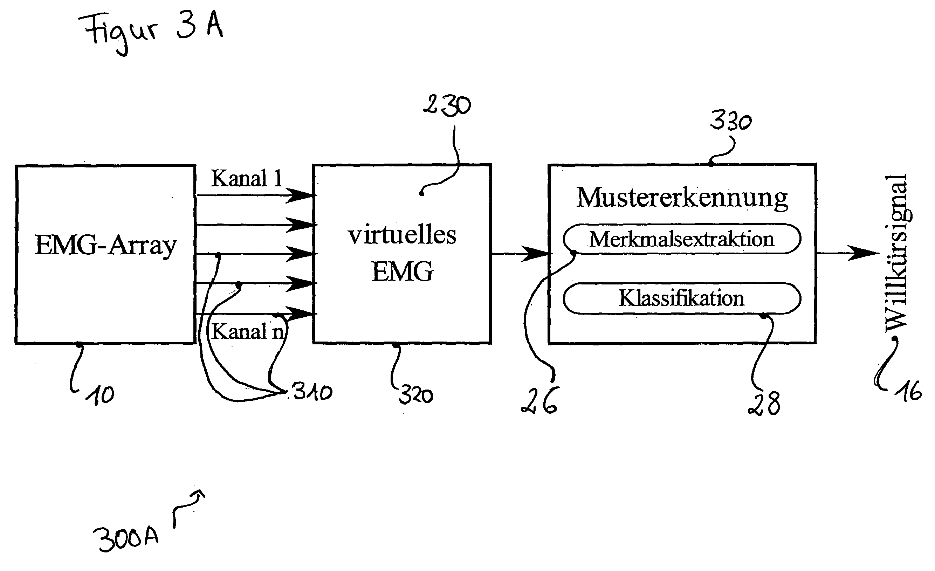

Figur 3a stellt einen Signalfluss 300A zur Verarbeitung von virtuellen EMG-Signalen dar. Die mit Hilfe eines Elektromyographiesensorgitters 10 bestimmten Muskelaktivitätssignale 212 werden über n Kanäle 310 einer Kombinationseinheit 320 zugeführt und dort zu einem virtuellen EMG-Signal 230 kombiniert. Das virtuelle EMG-Signal 230 durchläuft ein typisches Mustererkennungsverfahren 330 mit den Schritten Vorverarbeitung, Merkmalsextraktion 26, Merkmalreduktion und Klassifikation 28. Als Resultat der Mustererkennung 330 wird ein Willkürsignal 16 ausgegeben.

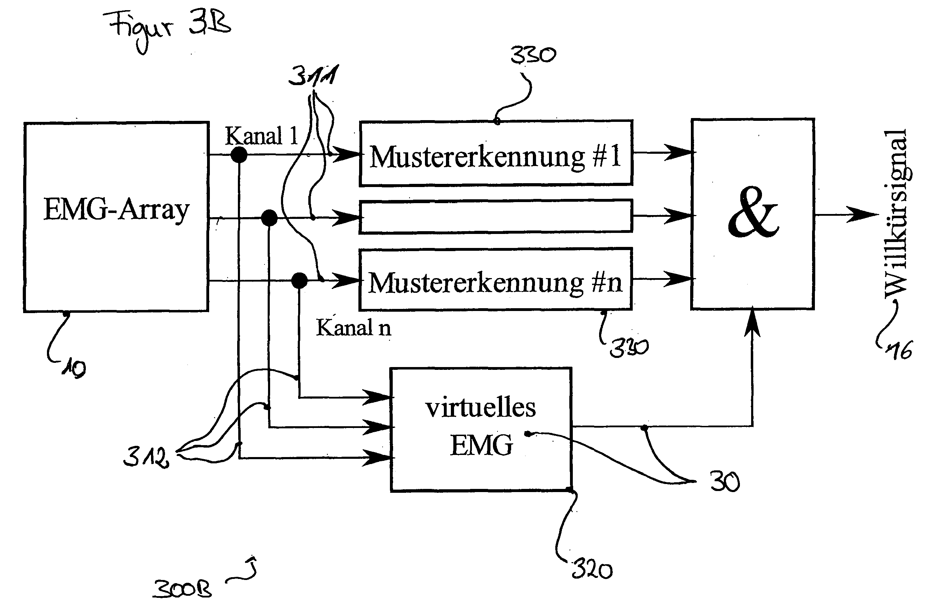

Figur 3b zeigt einen alternativen Signalfluss 300B für die Erzeugung eines Willkürsignals. Mit dem EMG-Array 10 werden Muskelaktivitätssignale 212 aufgenommen. Diese werden einzeln über n Kanäle 311 jeweils einer Mustererkennung 330 mit den Schritten Vorverarbeitung, Merkmalsextraktion, Reduktion und Klassifikation unterzogen. Gleichzeitig werden die Muskelaktivitätssignale 212 über n Kanäle 312 einer Kombinationseinheit 320 zugeführt und zu einem virtuellen EMG-Signal 230 kombiniert. Um das Willkürsignal 16 zu erhalten, werden die Ergebnisse der Mustererkennung 330 jedes einzelnen Muskelaktivitätssignals 212 sowie das virtuelle EMG-Signal 230 miteinander verarbeitet.

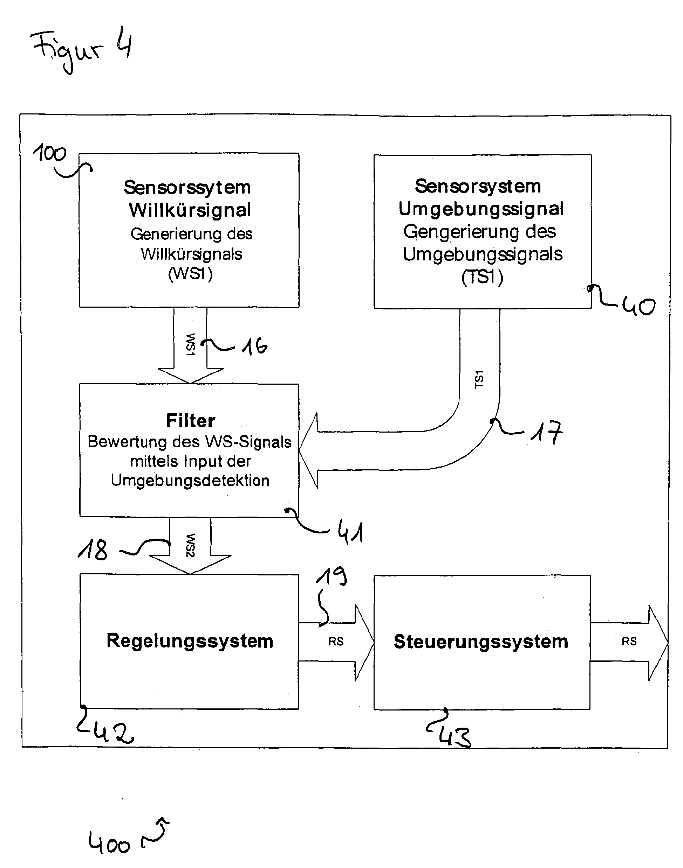

Figur 4 zeigt ein adaptives, umgebungsdetektierendes Steuerungs- und Regelsystem 400 für Prothesen mit Willkürsteuerung. Das System 400 weist ein System zur Generierung eines Willkürsignals, welches die Form des in Figur 1 dargestellten System 100 aufweist, auf, mit dessen Hilfe aus Muskelaktivitätssignalen 212 sowie Zustandsinformationen 220 ein Willkürsignal 16 generiert werden kann. Zusätzlich weist das Steuerungs- und Regelsystem 400 ein Sensorsystem 40 zur Aufnahme eines Umgebungssignals und zur Generierung von Umgebungssignalen 17 auf. In dem Steuerungs- und Regelsystem 400 werden einerseits das Willkürsignal 16 und andererseits das Umgebungssignal 17 an einen Filter 41 weitergeleitet, welcher eine Bewertung des Willkürsignals 16, welches auch als primäres Willkürsignal bezeichnet werden kann, mit Hilfe der Umgebungssignale 17 durchführt und als bewertetes Willkürsignal 18 (sekundäres Willkürsignal) an ein Regelungssystem 42 weitergibt. Das Regelungssystem 42 kann ein Regelsignal 19 an ein Steuersystem 43 weiterleiten, und somit für die Steuerung der Prothese sorgen.

Die vorliegende Erfindung geht dahingehend über den aktuellen Stand der Technik hinaus, dass die Willkürerkennung auch in dynamischen Bewegungen des Prothesenträgers bei verschiedenen Bewegungsformen möglich ist und die Bewegungssituationserkennung und die Willkürerkennung eine funktionale Einheit bilden. Zusätzlich zu dieser Funktion ist es möglich, die Umgebung zu detektieren und die Prothese auf mögliche Hindernisse vorzubereiten. Durch die automatische Anpassung an die Umgebung ist ein zusätzliches Sicherheitssystem bereitgestellt.

Durch die Kombination einer Willkürsteuerung und Bewegungserfassung mit einer Umgebungsdetektion ist es möglich, willkürliche Bewegungen mit einer Prothese in jedem Bewegungszustand durchzuführen, welche zusätzlich durch ein Sicherheitssystem bewertet werden. Ähnlich der Assistenzsysteme bei Fahrzeugen kann der Benutzer zwar die Anregung durch Willkür-Stimulation durchführen, trotzdem wird eine Fehlbenutzung durch den Prothesenträger verhindert.

## Patentansprüche

1. Verfahren zur adaptiven Steuerung und Regelung einer Prothese mit Willkürsteuerung, wobei
zunächst an einem Muskel eines Prothesenträgers eine Vielzahl an Muskelaktivitätssignalen gemessen wird,
gleichzeitig Zustandsinformationen zu einem tatsächlichen Bewegungszustand des Prothesenträgers gemessen werden, aus welchen mindestens ein möglicher, aktueller Bewegungszustand bestimmt wird,
danach aus den Muskelaktivitätssignalen mit Hilfe eines Verfahrens zur Detektion von Signalmustern Muskelaktivitätsmerkmale extrahiert werden,
anschließend aus den extrahierten Muskelaktivitätsmerkmalen mit Hilfe des mindestens einen möglichen, aktuellen Bewegungszustands mindestens ein Willkürsignal bestimmt wird, und
das Willkürsignal zur Auswertung und/oder zur Regelung und/oder Steuerung einer Aktorik der Prothese verwendet wird.

2. Verfahren nach dem vorhergehenden Anspruch, **dadurch gekennzeichnet, dass**
bei der Extraktion der Muskelaktivitätsmerkmale aus den Muskelaktivitätssignalen der mindestens eine mögliche, aktuelle Bewegungszustand berücksichtigt wird, und/oder dass
der mindestens eine mögliche, aktuelle Bewegungszustand ein Bewegungszustand ist, welcher mit einer einen Schwellwert überschreitenden Wahrscheinlichkeit einem tatsächlichen Bewegungszustand des Prothesenträgers entspricht.

3. Verfahren nach einem der vorhergehenden Ansprüche, **dadurch gekennzeichnet, dass**
die Vielzahl an Muskelaktivitätssignalen vor der Extraktion von Muskelaktivitätsmerkmalen zu virtuellen Muskelaktivitätssignalen kombiniert werden und dass aus den virtuellen Muskelaktivitätssignalen die Muskelaktivitätsmerkmale extrahiert werden.

4. Verfahren nach einem der vorhergehenden Ansprüche, **dadurch gekennzeichnet, dass**
die Vielzahl an Muskelaktivitätssignalen mittels mindestens einer ersten Erkennungseinheit zur Messung von Muskelaktivitätssignalengemessen wird und/oder dass
die Zustandsinformationen mittels einer zweiten Erkennungseinheit zur Messung von Zustandsinformationen zu einem tatsächlichen Bewegungszustand des Prothesenträgersgemessen werden.

5. Verfahren nach einem der vorhergehenden Ansprüche, **dadurch gekennzeichnet, dass**
während der Messung von Muskelaktivitätssignalen und von Zustandsinformationen außerdem mindestens ein Umgebungssignal gemessen wird.

6. Verfahren nach dem vorhergehenden Anspruch, **dadurch gekennzeichnet, dass** das Willkürsignal mit Hilfe des mindestens einen Umgebungssignals gefiltert und/oder bewertet wird.

7. Adaptives Steuerungs- und Regelsystem (400) für Prothesen mit Willkürsteuerung, mit einem Signalbestimmungssystem zur Bestimmung eines Willkürsignals, einem Regelungssystem zur Auswertung und/oder zur Regelung einer Aktorik einer Prothese mittels des Willkürsignals und zur Ausgabe eines Regelsignals (19) und einem Steuerungssystem (43) zur Ansteuerung der Aktorik der Prothese auf der Basis des Regelsignals, wobei das Signalbestimmungssystem mindestens eine erste Erkennungseinheit zur Messung einer Vielzahl an Muskelaktivitätssignalen (212) an einem Muskel eines Prothesenträgers; und
mindestens einen Prozessor zur Bestimmung von Muskelaktivitätsmerkmalen mit Hilfe eines Verfahrens zur Detektion von Signalmustern enthält,
**dadurch gekennzeichnet, dass**
das Signalbestimmungssystem außerdem mindestens eine zweite Erkennungseinheit zur Messung von Zustandsinformationen (220) zu einem tatsächlichen Bewegungszustand des Prothesenträgers aufweist, und dass
der Prozessor so ausgebildet ist, dass mit dem Prozessor aus den Zustandsinformationen mindestens ein möglicher, aktueller Bewegungszustand des Prothesenträgers bestimmbar ist, dass aus den Muskelaktivitätssignalen Muskelaktivitätsmerkmale extrahierbar sind und dass aus den extrahierten Merkmalen mit Hilfe des mindestens einen möglichen, aktuellen Bewegungszustands das mindestens eine Willkürsignal (16,18) bestimmbar ist.

8. Steuerungs- und Regelsystem nach dem vorhergehenden Anspruch, **dadurch gekennzeichnet, dass**
die mindestens eine erste Erkennungseinheit mindestens einen Sensor oder eine räumliche und verteilte Anordnung von Sensoren, ausgewählt aus der Gruppe enthaltend Elektrode, Drucksensor, Ultraschallsensor, Sensor, welcher eine Muskelkontraktion nicht-invasiv erkennen und messen kann, oder eine Koombination hiervon, aufweist und/oder dass
die mindestens eine zweite Erkennungseinheit mindestens einen Inertialsensor aufweist.

9. Steuerungs- und Regelsystem nach einem der beiden vorhergehenden Ansprüche, **dadurch gekennzeichnet, dass** der Prozessor enthält:
eine Vorverarbeitungseinheit zur Erzeugung mindestens eines virtuellen Muskelaktivitätssignals aus der Vielzahl an Muskelaktivitätssignalen und/oder zur Bestimmung des mindestens einen möglichen, aktuellen Bewegungszustands des Prothesenträgers, aus den Zustandsinformationen;
eine Merkmalsextraktionseinheit zur Gewinnung von Muskelaktivitätsmerkmalen aus dem mindestens einen virtuellen Muskelaktivitätssignal oder aus der Vielzahl an Muskelaktivitätssignalen, und
eine Klassifizierungseinheit zur Einteilung der Muskelaktivitätsmerkmale mittels des mindestens einen möglichen, aktuellen Bewegungszustandes in Klassen und zur Erzeugung des Willkürsignals.

10. Steuerungs- und Regelsystem nach einem der drei vorhergehenden Ansprüche, **dadurch gekennzeichnet, dass** das Bestimmungssystem mindestens eine dritte Erkennungseinheit zur Erfassung mindestens eines Umgebungssignalsaufweist, wobei das Umgebungssignal zur Filterung des Willkürsignals einsetzbar ist.

11. Steuerungs- und Regelsystem nach dem vorhergehenden Anspruch, **dadurch gekennzeichnet, dass** das Bestimmungssystem mindestens einen Filter zur Bewertung des Willkürsignals mittels des mindestens einen Umgebungssignals aufweist.

12. Prothese mit Willkürsteuerung, mit deren Hilfe ein Prothesenträger aktive Bewegungen ausführen kann, **dadurch gekennzeichnet, dass** die Prothese ein adaptives Steuerungs- und Regelsystem nach einem der Ansprüche 7 bis 11 aufweist.

13. Prothese nach dem vorhergehenden Anspruch, **dadurch gekennzeichnet, dass** die Prothese eine aktive oder passive Prothese ist.

14. Prothese nach einem der beiden vorhergehenden Ansprüche, **dadurch gekennzeichnet, dass** die Prothese mindestens ein zustandveränderbares Element aufweist.

## Claims

1. Method for the adaptive control and regulation of a prosthesis with voluntary control, wherein
firstly, a plurality of muscle activity signals are measured on a muscle of a prosthesis-wearer,
at the same time, status information regarding an actual movement status of the prosthesis-wearer is measured, from which at least one potential, current movement status is determined,
then, muscle activity features are extracted from the muscle activity signals with the aid of a method for the detection of signal patterns,
finally, at least one voluntary signal is determined from the extracted muscle activity features with the aid of the at least one potential, current movement status, and
the voluntary signal is used for the evaluation and/or for the regulation and/or control of an actuator of the prosthesis.

2. Method according to the preceding claim, **characterised in that**,
during the extraction of the muscle activity features from the muscle activity signals, the at least one potential, current movement status is considered, and/or
the at least one potential, current movement status is a movement status which corresponds to an actual movement status of the prosthesis-wearer with a level of probability exceeding a threshold value.

3. Method according to one of the preceding claims, **characterised in that**,
before the extraction of muscle activity features, the plurality of muscle activity signals are combined to form virtual muscle activity signals and the muscle activity features are extracted from the virtual muscle activity signals.

4. Method according to one of the preceding claims, **characterised in that**,
the plurality of muscle activity signals is measured by means of at least one first detection unit for the measurement of muscle activity signals, and/or
the status information is measured by means of a second detection unit for the measurement of status information regarding an actual movement status of the prosthesis-wearer.

5. Method according to one of the preceding claims, **characterised in that**,
at least one environmental signal is moreover measured during the measurement of muscle activity signals and status information.

6. Method according to the preceding claim, **characterised in that** the voluntary signal is filtered and/or evaluated with the aid of the at least one environmental signal.

7. Adaptive control and regulation system (400) for prostheses with voluntary control, having a signal determination system for determining a voluntary signal, a regulation system for evaluating and/or regulating an actuator of a prosthesis by means of the voluntary signal and for emitting a regulating signal (19), and a control system (43) for controlling the actuator of the prosthesis on the basis of the regulating signal, wherein the signal determination system contains:
at least one first detection unit for the measurement of a plurality of muscle activity signals (212) on a muscle of a prosthesis-wearer; and
at least one processor for the determination of muscle activity features with the aid of a method for the detection of signal patterns,
**characterised in that**,
the signal determination system moreover has at least one second detection unit for the measurement of status information (220) regarding an actual movement status of the prosthesis-wearer, and
the processor is designed in such a way that at least one potential, current movement status of the prosthesis-wearer is able to be determined with the processor from the status information,
muscle activity features are able to be extracted from the muscle activity signals, and
the at least one voluntary signal (16, 18) is able to be determined from the extracted features with the aid of the at least one potential, current movement status.

8. Control and regulation system according to the preceding claim, **characterised in that**,
the at least one first detection unit has at least one sensor or a spatial and distributed arrangement of sensors, selected from the group containing electrodes, pressure sensors, ultrasonic sensors, sensors that are able to detect and measure a muscle contraction non-invasively, or a combination thereof, and/or
the at least one second detection unit has at least one inertial sensor.

9. Control and regulation system according to one of the two preceding claims, **characterised in that** the processor contains:
a pre-processing unit for the generation of at least one virtual muscle activity signal from the plurality of muscle activity signals and/or for the determination of the at least one potential, current movement status of the prosthesis-wearer, from the status information;
a feature extraction unit for the extraction of muscle activity features from the at least one virtual muscle activity signal or from the plurality of muscle activity signals, and
a classification unit for the classification into categories of the muscle activity features by means of the at least one potential, current movement status and for the generation of the voluntary signal.

10. Control and regulation system according to one of the three preceding claims, **characterised in that** the determination system has at least one third detection unit for the capturing of at least one environmental signal, wherein the environmental signal is able to be used for filtering the voluntary signal.

11. Control and regulation system according to the preceding claim, **characterised in that** the determination system has at least one filter for the evaluation of the voluntary signal by means of the at least one environmental signal.

12. Prosthesis with voluntary control, with the aid of which a prosthesis-wearer is able to execute active movements, **characterised in that** the prosthesis has an adaptive control and regulation system according to one of claims 7 to 11.

13. Prosthesis according to the preceding claim, **characterised in that** the prosthesis is an active or passive prosthesis.

14. Prosthesis according to one of the two preceding claims, **characterised in that** the prosthesis has at least one state-alterable element.

## Revendications

1. Procédé de commande et de régulation adaptative d'une prothèse avec commande arbitraire,
une pluralité de signaux d'activité musculaire étant d'abord mesurée au niveau d'un muscle d'un support de prothèse,
des informations d'état concernant un état de mouvement effectif du support de prothèse étant simultanément mesurées, à partir desquelles au moins un état de mouvement actuel possible est déterminé,
des caractéristiques d'activité musculaire étant ensuite extraites des signaux d'activité musculaire à l'aide d'un procédé de détection de modèles de signaux,
au moins un signal arbitraire étant ensuite déterminé à partir des caractéristiques d'activité musculaire extraites à l'aide de l'au moins un état de mouvement actuel possible et
le signal arbitraire étant utilisé pour l'analyse et/ou la régulation et/ou la commande d'un ensemble d'actionneurs de la prothèse.

2. Procédé selon la revendication précédente, **caractérisé en ce que**
par l'extraction des caractéristiques d'activité musculaire des signaux d'activité musculaires, l'au moins un état de mouvement actuel possible est pris en compte et/ou **en ce que**
l'au moins un état de mouvement actuel possible est un état de mouvement qui correspond à une probabilité d'état de mouvement effectif du support de prothèse dépassant une valeur seuil.

3. Procédé selon l'une des revendications précédentes, **caractérisé en ce que**
la pluralité de signaux d'activité musculaire sont combinées, avant l'extraction de caractéristiques d'activité musculaire, avec des signaux d'activité musculaire virtuels et **en ce que** les caractéristiques d'activité musculaire sont extraites des signaux d'activité musculaire virtuels.

4. Procédé selon l'une des revendications précédentes, **caractérisé en ce que**
la pluralité de signaux d'activité musculaire est mesurée au moyen d'une première unité de détection pour la mesure de signaux d'activité musculaire et/ou **en ce que**
les informations d'état sont mesurées au moyen d'une deuxième unité de détection pour la mesure d'informations d'état en un état de mouvement effectif du support de prothèse.

5. Procédé selon l'une des revendications précédentes, **caractérisé en ce que**
pendant la mesure des signaux d'activité musculaire et des informations d'état, un signal d'environnement est en outre mesuré.

6. Procédé selon la revendication précédente, **caractérisé en ce que** le signal arbitraire est filtré et/ou évalué à l'aide de l'au moins un signal d'environnement.

7. Système de commande et de régulation adaptatif (400) pour prothèses avec commande arbitraire, avec un système de détermination de signaux pour la détermination d'un signal arbitraire,
un système de régulation pour l'analyse et/ou la régulation d'un ensemble d'actionneurs d'une prothèse au moyen du signal arbitraire et pour l'émission d'un signal de régulation (19), et
un système de commande (43) pour la commande des actionneurs de la prothèse sur la base du signal de régulation, le système de détermination de signaux comprenant au moins une première unité de détection pour la mesure d'une pluralité de signaux d'activité musculaire (212) au niveau d'un muscle d'un support de prothèse ; et
au moins un processeur pour la détermination de caractéristiques d'activité musculaire à l'aide d'un procédé de détection de modèles de signaux,
**caractérisé en ce que**
le système de détermination de signaux comprend en outre au moins une deuxième unité de détection pour la mesure d'informations d'état (220) en un état de mouvement effectif du support de prothèse et **en ce que** le processeur est conçu de façon à ce que, avec le processeur, à partir des informations d'état, au moins un état de mouvement actuel possible du support de prothèse peut être déterminé, de façon à ce que, à partir des signaux d'activité musculaire, des caractéristiques d'activité musculaire puissent être extraites et de façon à ce que l'au moins un signal arbitraire (16, 18) puisse être déterminé à partir des caractéristiques extraites, à l'aide de l'au moins un état de mouvement actuel possible.

8. Système de commande et de régulation selon la revendication précédente, **caractérisé en ce que**
l'au moins une première unité de détection comprend au moins un capteur ou une disposition spatiale et répartie de capteurs sélectionnés parmi le groupe comprenant : une électrode, un capteur de pression, un capteur d'ultrasons, un capteur qui détecte et peut mesurer une contraction musculaire de manière non invasive, ou une combinaison de ceux-ci et/ou en ce que
l'au moins une deuxième unité de détection comprend au moins un capteur inertiel.

9. Système de commande et de régulation selon l'une des deux revendications précédentes, **caractérisé en ce que** le processeur comprend :
une unité de prétraitement pour la génération d'au moins un signal d'activité musculaire virtuel à partir de la pluralité de signaux d'activité musculaire et/ou pour la détermination de l'au moins un état de mouvement actuel possible du support de prothèse, à partir des informations d'état ;
une unité d'extraction de caractéristiques pour l'extraction de caractéristiques d'activité musculaire à partir de l'au moins un signal d'activité musculaire virtuel ou à partir de la pluralité de signaux d'activité musculaire et
une unité de classification pour la classification des caractéristiques d'activité musculaire en classes à l'aide de l'au moins un état de mouvement actuel possible et pour la génération du signal arbitraire.

10. Système de commande et de régulation selon l'une des trois revendications précédentes, **caractérisé en ce que** le système de détermination comprend au moins une troisième unité de détection pour la détection d'au moins un signal d'environnement, le signal d'environnement pouvant être utilisé pour filtrer le signal arbitraire.

11. Système de commande et de régulation selon la revendication précédente, **caractérisé en ce que** le système de détermination comprend au moins un filtre pour l'évaluation du signal arbitraire à l'aide de l'au moins un signal d'environnement.

12. Prothèse avec commande arbitraire à l'aide de laquelle un support de prothèse peut effectuer des mouvements actifs, **caractérisée en ce que** la prothèse comprend un système de commande et de régulation adaptatif selon l'une des revendications 7 à 11.

13. Prothèse selon la revendication précédente, **caractérisée en ce que** la prothèse est une prothèse active ou passive.

14. Prothèse selon l'une des deux revendications, **caractérisée en ce que** la prothèse comprend au moins un élément dont l'état est variable.
